# EUROPÄISCHE PATENTANMELDUNG

(11) **EP 3 895 677 A1**
(43) Veröffentlichungstag der Anmeldung: **20.10.2021**
(21) Anmeldenummer: 20169969.1
(22) Anmeldetag: 16.04.2020
(51) Int. Cl.: A61F 17/00

(54) **NOTFALLKAPSEL MIT NOTFALLEQUIPMENT UND VERFAHREN ZUM BEFÜLLEN DER NOTFALLKAPSEL**

(71) Anmelder: Notfall Akademie Schweiz AG, 8840 Einsiedeln (CH)
(72) Erfinder: Janssen, Sascha, 8840 Einsiedeln (CH)
(74) Vertreter: Nückel, Thomas

(57) **Zusammenfassung**

Die erfindungsgemässe Notfallkapsel mit Notfallequipment umfasst einen manuell öffenbaren Hohlkörper (11, 12). Zudem ist ein Primärpackmittel (4) aus flexiblem Material vorgesehen, das einen Stauraum (4.3) aufweist, in dem sich das Notfallequipment (1, 2, 3) befindet. Das Primärpackmittel (4) ist derart ausgebildet, dass dessen Stauraum (4.3) auf ein definiertes Packmass verkleinerbar ist und das Packmass auch ausserhalb des Hohlkörpers (11, 12) beibehält. Des Weiteren ist vorgesehen, dass das Primärpackmittel (4) samt Notfallequipment (1, 2, 3) im verkleinerten Zustand im Hohlkörper (11, 12) angeordnet ist.

## Beschreibung

### Technisches Gebiet

Die Erfindung betrifft eine Notfallkapsel mit Notfallequipment und ein Verfahren zum Befüllen der Notfallkapsel mit Notfallequipment.

Wenn eine Person aufgrund eines Notfalls medizinische Hilfe benötigt, ist es die Pflicht des Ersthelfers / der Ersthelferin dieser Person umgehend Erste Hilfe zu leisten. Es ist jedoch darauf zu achten, dass sich der Ersthelfer / die Ersthelferin bei der Hilfe ausreichend gegen eventuelle gesundheitliche Risiken schützen kann. Insbesondere soll die Gefahr einer Infektion wirksam reduzierten werden, die vom Kontakt mit der hilfsbedürftigen Person, deren Körperflüssigkeiten, Blut und Atem ausgehen kann. Hierzu wird dem Helfer / der Helferin empfohlen, Handschuhe zu tragen und gegebenenfalls der hilfsbedürftigen Person eine Maske anzulegen. Das in der erfindungsgemässen Notfallkapsel enthaltene Notfallequipment stellt die für den Helfenden / die Helfende erforderliche Schutzausrüstung zur Verfügung.

### Stand der Technik

Eine Kapsel, die Notfallequipment enthält, ist von der Firma Notfall Akademie Schweiz AG über deren Webseite https://www.notfallakademie.ch/shop/notfallkapsel/ käuflich erwerbbar. Bei dieser Kapsel besteht das Notfallequipment aus zwei Handschuhen, einer Beatmungsmaske und einer Anleitung, zum Beispiel für die Reanimation und um den Patienten bei Bewusstlosigkeit und bestehender Eigenatmung in die stabile Seitenlage zu bringen. Diese Teile werden im Folgenden auch als Schutzgegenstände bezeichnet. Die Kapsel hat den Nachteil, dass das Befüllen der Kapsel mit dem Notfallequipment ausserordentlich zeitaufwändig ist. Zuerst müssen die beiden Handschuhe von Hand zusammengelegt werden. Auch die Beatmungsmaske und die Anleitung müssen jeweils separat zuerst zusammengelegt werden. Erst dann kann eine der beiden Kapselschalen mit den zusammengelegten Handschuhen, der Beatmungsmaske und der Anleitung - wiederum von Hand - befüllt werden. Dabei kann es immer wieder vorkommen, dass aufgrund von Unachtsamkeit und/oder mangelnder Fingerfertigkeit das Notfallequipment nicht vollständig in die Kapselschale passt. Dann müssen die Schutzgegenstände wieder aus der Kapsel herausgenommen, erneut sauber zusammengelegt und wieder in die Kapselschale eingeführt werden. Es kann auch vorkommen, dass Teile der Schutzgegenstände beim Zusammenstecken der beiden Kapselschalen zwischen die Kapselschalen geraten und verhindern, dass sich die Kapselschalen zusammenstecken lassen oder aus der Kapsel herausragen. Wenn dies der Fall ist, besteht zudem die Gefahr, dass der oder die Schutzgegenstände beschädigt werden.

### Darstellung der Erfindung

Eine Aufgabe der Erfindung ist es, eine Notfallkapsel mit Notfallequipment anzugeben, wobei die Abmessungen der Notfallkapsel so klein wie möglich sind und zugleich ein möglichst umfassendes Notfallequipment in der Notfallkapsel Platz findet.

Ein Vorteil der erfindungsgemässen Lösung ist, dass die Notfallkapsel deutlich schneller als die Kapsel beim Stand der Technik mit dem Notfallequipment bestückt werden kann. Zudem wird bei der erfindungsgemässen Lösung die Fehlerquote beim Bestücken reduziert.

Vorteilhafter Weise ist die erfindungsgemässe Notfallkapsel so klein, dass sie von jedermann jederzeit und überall hin mitgenommen werden kann.

Eine weitere Aufgabe der Erfindung ist es, ein Verfahren zum Befüllen der Notfallkapsel anzugeben, das es trotz der kleinen Abmessungen der Notfallkapsel ermöglicht, das erforderliche Notfallequipment einfach, schnell und fehlerfrei in der Notfallkapsel unterbringen zu können.

Die Aufgabe wird durch eine Notfallkapsel mit Notfallequipment mit den in Patentanspruch 1 angegebenen Merkmalen gelöst.

Die erfindungsgemässe Notfallkapsel mit Notfallequipment umfasst einen manuell öffenbaren Hohlkörper. Zudem ist ein Primärpackmittel aus flexiblem Material vorgesehen, das einen Stauraum aufweist, in dem sich das Notfallequipment befindet. Das Primärpackmittel ist derart ausgebildet, dass dessen Stauraum auf ein definiertes Packmass verkleinerbar ist und das Packmass auch ausserhalb des Hohlkörpers beibehält. Des Weiteren ist vorgesehen, dass das Primärpackmittel samt Notfallequipment im verkleinerten Zustand im Hohlkörper angeordnet ist.

Die Aufgabe wird zudem durch ein Verfahren zum Befüllen der Notfallkapsel mit Notfallequipment mit den in Patentanspruch 12 angegebenen Merkmalen gelöst.

Das erfindungsgemässe Verfahren zum Befüllen der oben beschriebenen Notfallkapsel mit Notfallequipment umfasst folgende Schritte. In den Stauraum des Primärpackmittels wird das Notfallequipment eingeführt. Der Stauraum des Primärpackmittels wird verkleinert und anschliessend wird das das Notfallequipment enthaltende, verkleinerte Primärpackmittel im Hohlkörper der Notfallkapsel angeordnet.

Vorteilhafte Weiterbildungen der Erfindung ergeben sich aus den in den abhängigen Patentansprüchen angegebenen Merkmalen.

Bei einer Ausführungsform der erfindungsgemässen Notfallkapsel ist das Primärpackmittel derart ausgebildet, dass dessen Stauraum durch Pressen, durch Unterdruck und/oder durch Erhitzen verkleinerbar ist. Durch diesen Vorgang wird der Stauraum auf das gewünschte Packmass reduziert und behält dieses Packmass dann dauerhaft.

Bei einer anderen Ausführungsform der erfindungsgemässen Notfallkapsel ist das Primärpackmittel aus Kunststoff hergestellt.

Bei einer weiteren Ausführungsform der erfindungsgemässen Notfallkapsel weist das Primärpackmittel eine Einfüllöffnung auf, die verschweisst oder verklebt ist, wenn der Stauraum mit dem Notfallequipment befüllt und auf das gewünschte Packmass reduziert ist. Dies hat den Vorteil, dass das Notfallequipment noch besser vor Schmutz geschützt ist.

Die Einfüllöffnung des Primärpackmittels nach dem Verkleinern luftdicht zu verschweissen oder zu verkleben hat folgenden Vorteil. Wenn im Stauraum des Primärpackmittels ein Unterdruck erzeugt wird, um es auf das gewünschte kleine Packmass zu bringen, sorgt der Unterdruck im luftdicht verschlossenen Stauraum dafür, dass das Primärpackmittel formhaltig ist und bleibt. Das Primärpackmittel mit dem Notfallequipment kann so zwischengelagert und dann später bei Bedarf in den Hohlkörper eingesetzt werden. Auch kann eine Notfallkapsel deren Notfallequipment bereits einmal entnommen worden ist, beliebig oft wieder mit einem neuen Notfallequipment bestückt werden. Damit ist es auch möglich die leere Notfallkapsel getrennt vom Primärpackmittel mit dem Notfallequipment zu lagern und zu verkaufen.

Bei einer Weiterbildung der erfindungsgemässen Notfallkapsel ist das Primärpackmittel aus einem Kunststoff hergestellt, der bei Erwärmung schrumpft.

Bei einer zusätzlichen Weiterbildung der erfindungsgemässen Notfallkapsel weist der Hohlkörper eine erste Schale und eine zweite Schale auf. Die erste Schale und die zweite Schale sind formschlüssig miteinander verbindbar.

Es ist von Vorteil, wenn bei der erfindungsgemässen Notfallkapsel die erste Schale eine Nut und die zweite Schale einen Wulst aufweist. Der Wulst und die Nut sind dazu vorgesehen einen Formschluss zu bilden. Damit kann auf einfache Weise eine Schnappverbindung realisiert werden.

Es ist möglich, bei der erfindungsgemässen Notfallkapsel vorzusehen, dass die erste Schale und die zweite Schale miteinander verschraubt sind.

Bei der erfindungsgemässen Notfallkapsel kann das Notfallequipment Handschuhe und eine Maske umfassen.

Bei der erfindungsgemässen Notfallkapsel kann das Notfallequipment zudem eine Anleitung umfassen. Mit der Anleitung ist der Ersthelfer noch besser für eine adäquate Erste Hilfe gerüstet.

Es kann bei der erfindungsgemässen Notfallkapsel auch ein Aufhänger vorgesehen sein, der am Hohlkörper befestigt ist. Dies hat den Vorteil, dass man die Notfallkapsel einfach und schnell an den verschiedensten Gegenständen befestigen kann. So kann der Aufhänger zum Beispiel benutzt werden, um die Notfallkapsel an einem Schlüsselbund zu befestigen.

Bei einer Weiterbildung des erfindungsgemässen Verfahrens zum Befüllen der Notfallkapsel wird der Stauraum verkleinert, indem im Stauraum ein Unterdruck erzeugt wird. Vorteilhafter Weise wird der Stauraum danach luftdicht verschlossen, sodass der Unterdruck im Stauraum erhalten bleibt.

Bei einer anderen Weiterbildung des erfindungsgemässen Verfahrens zum Befüllen der Notfallkapsel wird der Stauraum verkleinert, indem das Primärpackmittel zusammengepresst wird. Mit diesem Vorgang kann zum Beispiel das Vakuumieren oder das Schrumpfen des Packmittels unterstütz werden.

Bei einer zusätzlichen Weiterbildung des erfindungsgemässen Verfahrens zum Befüllen der Notfallkapsel wird der Stauraum verkleinert, indem das Primärpackmittel durch Erhitzten einem Schrumpfungsprozess unterzogen wird. Nach dem Schrumpfvorgang bleibt das Primärpackmittel dauerhaft formhaltig.

Schliesslich kann bei dem erfindungsgemässen Verfahren zum Befüllen der Notfallkapsel vorgesehen sein, dass der Stauraum des Primärpackmittels verschlossen wird.

### Kurze Beschreibung der Zeichnungen

Im Folgenden wird die Erfindung mit mehreren Ausführungsbeispielen anhand von 24 Figuren weiter erläutert.
- Figur 1: zeigt einen Handschuh, der Teil des Notfallequipments sein kann, in der Draufsicht.
- Figur 2: zeigt eine Beatmungsmaske, die Teil des Notfallequipments sein kann.
- Figur 3: zeigt zwei Blätter einer Anleitung, die Teil des Notfallequipments sein kann, in einer dreidimensionalen Ansicht.
- Figur 4a: zeigt eine erste Ausführungsform der Primärverpackung, in der sich das Notfallequipment befinden kann, in der Draufsicht.
- Figur 4b: zeigt eine zweite Ausführungsform des Primärverpackung, in der sich das Notfallequipment befinden kann, in der Draufsicht.
- Figur 5a: zeigt eine erste Ausführungsform der erfindungsgemässen Notfallkapsel in einer dreidimensionalen Ansicht.
- Figur 5b: zeigt die erste Ausführungsform der mit dem Notfallequipment befüllten Notfallkapsel teilweise im Schnitt in einer dreidimensionalen Ansicht.
- Figur 6: zeigt die erste Ausführungsform der erfindungsgemässen Notfallkapsel in einer zweiten dreidimensionalen Ansicht.
- Figur 7: zeigt die erste Ausführungsform der erfindungsgemässen Notfallkapsel in einer dreidimensionalen Ansicht schräg von unten.
- Figur 8: zeigt die erste Ausführungsform der erfindungsgemässen Notfallkapsel in einer dreidimensionalen Ansicht teilweise im Schnitt ohne Notfallequipment.
- Figur 9: zeigt eine zweite Ausführungsform der erfindungsgemässen Notfallkapsel in einer dreidimensionalen Ansicht.
- Figur 10: zeigt die zweite Ausführungsform der erfindungsgemässen Notfallkapsel in der Ansicht von unten.
- Figur 11: zeigt die zweite Ausführungsform der erfindungsgemässen Notfallkapsel in einer dreidimensionalen Ansicht teilweise im Schnitt.
- Figur 12: zeigt eine dritte Ausführungsform der erfindungsgemässen Notfallkapsel in einer dreidimensionalen Ansicht.
- Figur 13: zeigt die dritte Ausführungsform der erfindungsgemässen Notfallkapsel in der Ansicht von unten.
- Figur 14: zeigt eine vierte Ausführungsform der erfindungsgemässen Notfallkapsel in einer dreidimensionalen Ansicht.
- Figur 15: zeigt die vierte Ausführungsform der erfindungsgemässen Notfallkapsel in der Seitenansicht.
- Figur 16: zeigt die vierte Ausführungsform der erfindungsgemässen Notfallkapsel in der Draufsicht.
- Figur 17: zeigt die vierte Ausführungsform der erfindungsgemässen Notfallkapsel in einer dreidimensionalen Ansicht teilweise im Schnitt.
- Figur 18: zeigt eine fünfte Ausführungsform der erfindungsgemässen Notfallkapsel in der Seitenansicht.
- Figur 19: zeigt eine sechste Ausführungsform der erfindungsgemässen Notfallkapsel in einer dreidimensionalen Ansicht.
- Figur 20: zeigt die sechste Ausführungsform der erfindungsgemässen Notfallkapsel in einer dreidimensionalen Ansicht ohne Aufkleber.
- Figur 21: zeigt die sechste Ausführungsform der erfindungsgemässen Notfallkapsel in der Draufsicht.
- Figur 22: zeigt die sechste Ausführungsform der erfindungsgemässen Notfallkapsel in der Seitenansicht.
- Figur 23: zeigt die sechste Ausführungsform der erfindungsgemässen Notfallkapsel Ansicht von unten.
- Figur 24: zeigt die sechste Ausführungsform der erfindungsgemässen Notfallkapsel in einer dreidimensionalen Ansicht teilweise im Schnitt.

### Wege zur Ausführung der Erfindung

Die Notfallkapsel umfasst einen manuell öffenbaren Hohlkörper zur Aufnahme und Aufbewahrung von Notfallequipment.

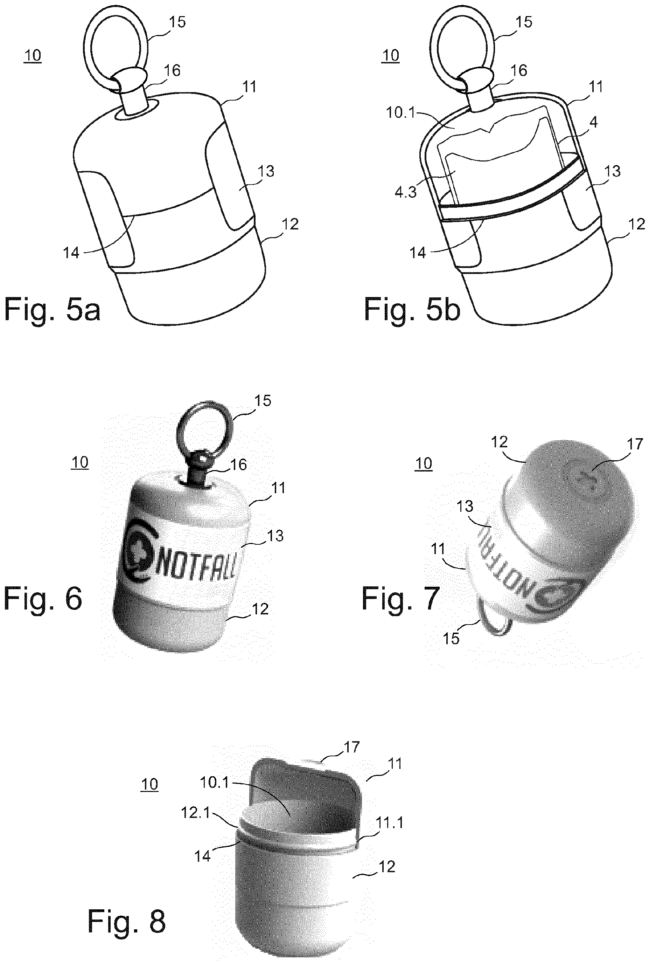

In den Figuren 5a, 5b, 6, 7 und 8 ist eine erste mögliche Ausführungsform der erfindungsgemässen Notfallkapsel 10 in verschiedenen Ansichten gezeigt. Bei dieser Ausführungsform umfasst die Notfallkapsel 10 eine erste Schale 11 und eine zweite Schale 12, die zusammen den manuell öffenbaren Hohlkörper bilden. Die beiden Schalen 11 und 12 sind mittels einer Schnappverbindung miteinander verbindbar und manuell, also ohne dass es Werkzeug bedarf, auch wieder voneinander trennbar. Die Schnappverbindung ist so ausgebildet, dass die beiden Schalen 11 und 12 von Hand wieder voneinander trennbar sind. Zur Bildung der Schnappverbindung ist auf der Innenseite der ersten Schale 11 eine Nut 11.1 und auf der Aussenseite der zweiten Schale 12 ein Wulst 12.1 angeordnet. Wenn die beiden Schalen 11 und 12 zusammengefügt sind, bilden der Wulst 12.1 und die Nut 11.1 eine formschlüssige Verbindung. Der Vorteil dieser Verbindung ist, dass sie schnell und einfach zu öffnen ist. Dies ist insbesondere im Notfall hilfreich, wenn also Eile geboten ist und keine Zeit zum Ausprobieren bleibt.

Im Inneren des Hohlkörpers steht ein Hohlraum 10.1 zur Aufnahme des Notfallequipments zur Verfügung. Das Notfallequipment umfasst vorzugsweise zwei Handschuhe 1 und eine Maske 2. Eine Anleitung 3 kann ebenfalls Teil des Notfallequipments sein.

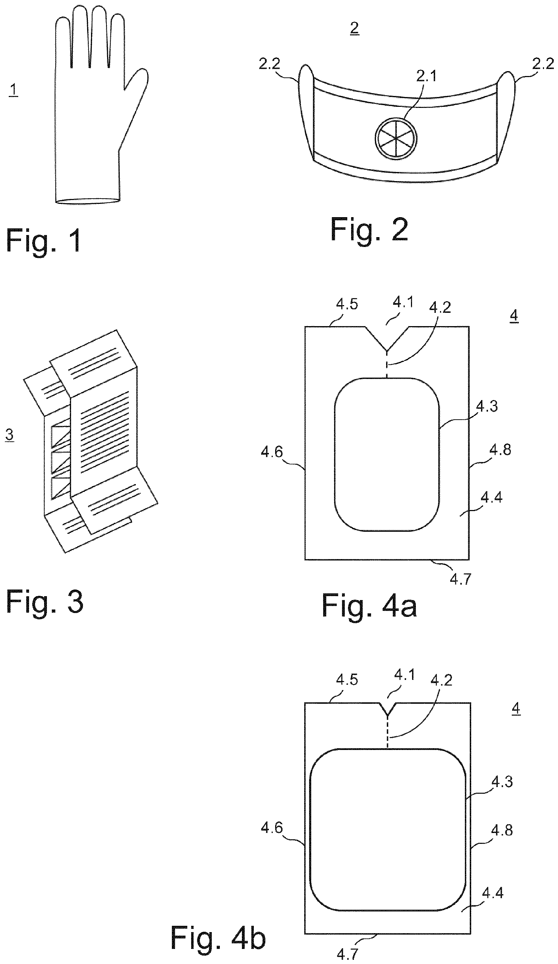

Eine mögliche Ausgestaltung eines Handschuhs 1 ist in Figur 1 gezeigt. Die Handschuhe 1 sind vorzugsweise puderfrei und aus Nitril hergestellt. Sie können aber auch aus Latex oder aus Vinyl hergestellt sein. Handschuhe aus Nitril haben den Vorteil, dass sie sehr elastisch und reissfest sind und von den meisten Allergikern bedenkenlos getragen werden können. Auch bei Kontakt mit Öl oder Chemikalien sind Handschuhe aus Nitril besser geeignet als solche aus Latex oder Vinyl. Zudem enthalten Nitrilhandschuhe keine Weichmacher und das Tastempfinden ist besser als bei Latex- oder Vinylhandschuhen. Werden die Handschuhe 1 vakuumverpackt, erhöht sich deren Haltbarkeit, sie trocknen langsamer aus.

Eine mögliche Ausgestaltung der Maske 2 ist in Figur 2 gezeigt. Bei dieser Ausgestaltung ist die Maske 2 eine Beatmungsmaske mit einem Filter 2.1, über den der Ersthelfer den Patienten beatmen kann. Die Maske 2 ist vorzugsweise aus einer Plastikfolie hergestellt und umfasst zudem zwei Elastikbänder 2.2, um die Maske am Kopf des Patienten fixieren zu können. Die beiden Elastikbänder 2.2 sind aber nicht zwingend erforderlich. Stattdessen oder zusätzlich dazu kann die Maske 2 auch eine Nasenklammer aufweisen (in den Figuren nicht gezeigt). Die Nasenklammer wird auf das Nasenbein geschoben und fixiert so die Maske. Die Maske 2 wird dem Patienten angelegt.

Mit der als Beatmungsmaske ausgebildeten Maske 2 kann sich der Ersthelfer / die Ersthelferin bei der Mund-zu-Mund-Beatmung schützen und verhindern, dass er/sie sich mit Körperflüssigkeiten oder Blut des Patienten kontaminiert.

In Figur 3 ist die Anleitung 3 symbolisch dargestellt. Die Anleitung 3 kann ein oder mehrere Blätter umfassen, auf denen verschiedene Vorgehensweisen zur Ersten Hilfe in Textform und/oder mit Piktogrammen erläutert sind. Insbesondere die Vorgehensweisen für lebensrettende und gesundheitserhaltende Sofortmassnahmen, wie zum Beispiel bei Atem- oder Kreislaufstillstand, können hier beschrieben sein. Auch die Anwendung der Maske 2 kann in der Anleitung 3 erklärt sein.

Die Anleitung 3 kann zum Beispiel eine Anleitung für die Reanimation enthalten. Damit kann der Ersthelfer / die Ersthelferin die ersten wichtigen Schritte einleiten, um dem Patienten zu helfen und um die Zeit bis zum Eintreffen der Rettungskräfte zu überbrücken.

Die Anleitung 3 kann auch die wichtigsten Informationen für eine stabile Seitenlage enthalten. Diese können zum Beispiel bildlich darstellt sein.

Bei der in den Figuren 5a, 5b, 6, 7 und 8 dargestellten ersten Ausführungsform der Notfallkapsel 10 ist auf der Aussenseite der Notfallkapsel 10 ein Aufkleber 13 angebracht. Auf dem Aufkleber 13 können sich verschiedene Informationen wie Text und/oder grafische Darstellungen befinden. Der Aufkleber 13 kann, wie in Fig. 5a gezeigt, so angebracht sein, dass er zumindest teilweise über die Fuge 14, die die Trennstelle zwischen den beiden Schalen 11 und 12 bildet, geklebt ist. Wenn die Notfallkapsel 10 geöffnet wird, reisst der Aufkleber 13 auseinander. Der Aufkleber 13 kann somit auch als Siegel dienen, um zu kennzeichnen, ob die Notfallkapsel bereits einmal geöffnet wurde.

Ein Ring 15 kann über eine Befestigung 16 an einer der beiden Schalen befestigt sein, in den Figuren ist dies die Schale 11. Der Ring 15 und die Befestigung 16 bilden einen Anhänger, der dazu dienen kann, die Notfallkapsel 10 an einem Gegenstand, wie zum Beispiel einem Schlüsselbund zu befestigen.

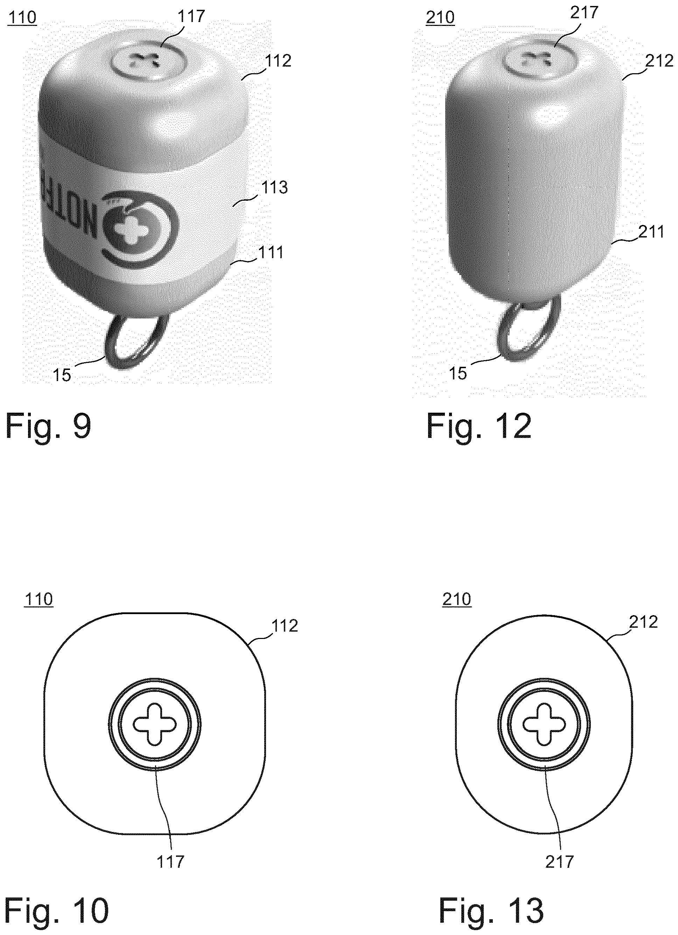

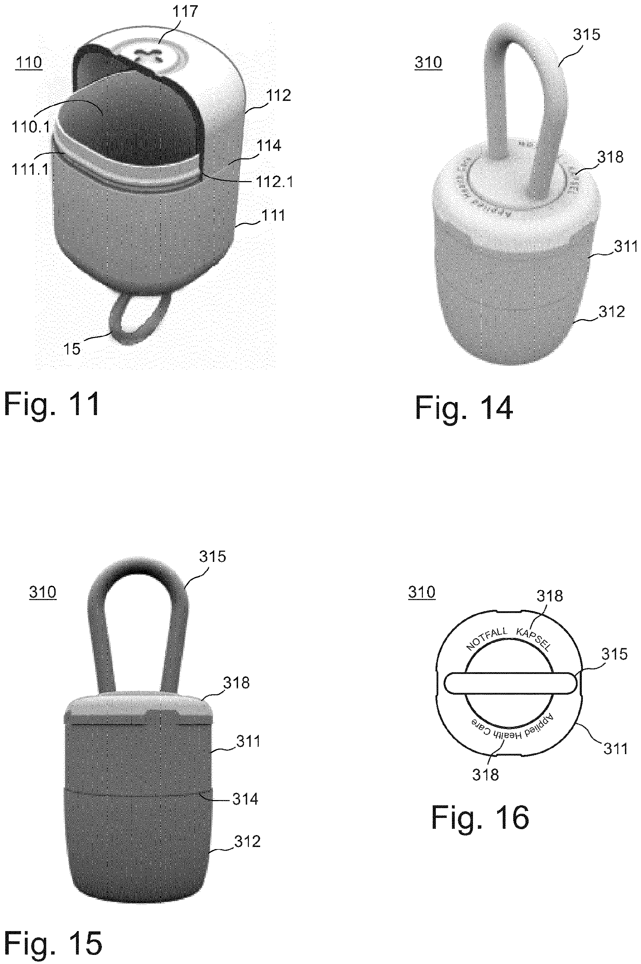

In den Figuren 9, 10 und 11 ist eine zweite mögliche Ausführungsform der erfindungsgemässen Notfallkapsel 110 in verschiedenen Ansichten gezeigt. Figur 9 zeigt die Notfallkapsel 110 in einer dreidimensionalen Ansicht. Figur 10 zeigt die Notfallkapsel 110 in der Ansicht von unten und Figur 11 zeigt die Notfallkapsel 110 ebenfalls in einer dreidimensionalen Ansicht, wobei die Schale 112 teilweise im Schnitt dargestellt ist. Die Notfallkapsel 110 unterscheidet sich von der Notfallkapsel 10 im wesentlichen im Querschnitt. Während die Querschnitte der Schalen 11 und 12 der Notfallkapsel 10 kreisrund sind, sind jene der Schalen 111 und 112 der Notfallkapsel 110 quadratisch mit abgerundeten Ecken. Am besten ist dies in Figur 10 zu erkennen. Auf der Unterseite der Schale 112 kann wie bei der Notfallkapsel 10 eine Prägung 117 vorgesehen sein.

In den Figuren 12 und 13 ist eine dritte mögliche Ausführungsform der erfindungsgemässen Notfallkapsel 210 in verschiedenen Ansichten gezeigt. Figur 12 zeigt die Notfallkapsel 210 in einer dreidimensionalen Ansicht. Figur 13 zeigt die Notfallkapsel 210 in der Ansicht von unten. Die Notfallkapsel 210 unterscheidet sich von der Notfallkapsel 110 im Querschnitt. Die Querschnitte der Schalen 211 und 212 der Notfallkapsel 210 sind eher rechteckig mit abgerundeten Ecken. Am besten ist dies in Figur 13 zu erkennen. Am Boden der Schale 212 kann eine Prägung 217 vorhanden sein.

In den Figuren 14, 15, 16 und 17 ist eine vierte mögliche Ausführungsform der erfindungsgemässen Notfallkapsel 310 in verschiedenen Ansichten gezeigt. Figur 145zeigt die Notfallkapsel 310 in einer dreidimensionalen Ansicht.

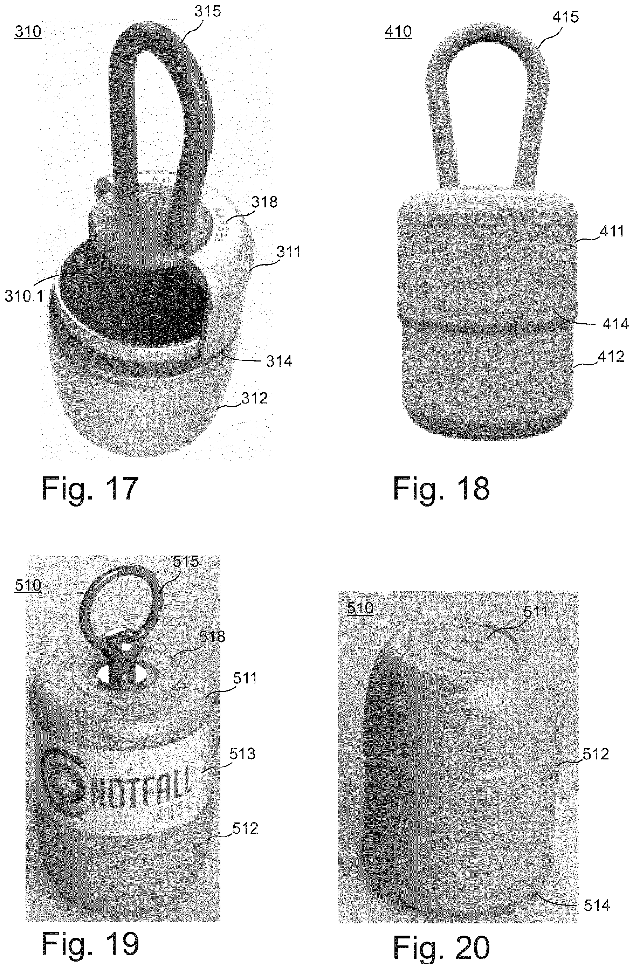

Figur 14 zeigt die Notfallkapsel 310 in der Seitenansicht. In Figur 16 ist die Notfallkapsel 310 in der Draufsicht dargestellt. Figur 17 zeigt die Notfallkapsel 310 in einer dreidimensionalen Ansicht, wobei die Schale 311 teilweise im Schnitt gezeigt ist. Auch bei dieser Ausführungsform sind zwei Schalen 311 und 312 vorgesehen. Deren Funktion ist dieselbe wie die der Schalen 11 und 12. Die Notfallkapsel 310 unterscheidet sich von den anderen Ausführungsformen der Notfallkapsel in der Form der Schalen 311 und 312 sowie in der Ausgestaltung des Aufhängers 315. Der Aufhänger 315 ist einstückig ausgebildet. Je nach Bedarf kann der Aufhänger 315 aus einem starren oder einem flexiblen Material hergestellt sein. Die Querschnitte der Schalen 311 und 312 der Notfallkapsel 310 sind im Wesentlichen kreisrund. Auf der Oberseite der Schale 311 kann Text 318 aufgedruckt oder eingeprägt sein. Im Hohlraum 311.1 ist das Notfallequipment (nicht gezeigt) angeordnet. Das Bezugszeichen 314 kennzeichnet die Trennlinie beziehungsweise Fuge zwischen den beiden Schalen 311 und 312.

In Figur 18 ist eine fünfte mögliche Ausführungsform der erfindungsgemässen Notfallkapsel 410 in der Seitenansicht gezeigt. Die Notfallkapsel 410 unterscheidet sich von den Notfallkapsel 310 in der Form der Schalen 411 und 412. Gegenüber der Notfallkapsel 310 ist die Notfallkapsel 310 tonnenförmiger ausgebildet. Das Bezugszeichen 414 kennzeichnet die Trennlinie beziehungsweise Fuge zwischen den beiden Schalen 411 und 412.

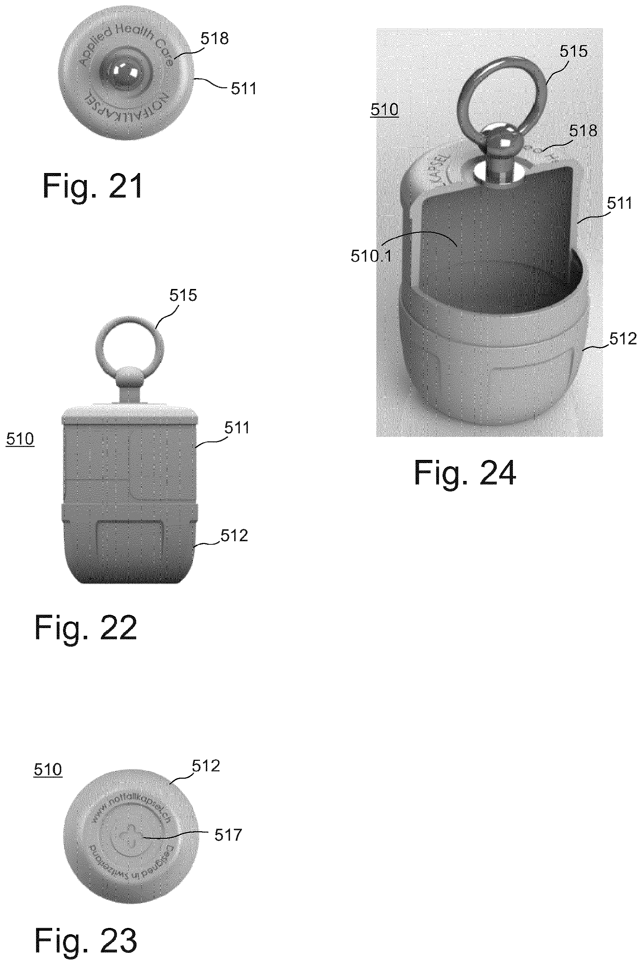

In Figur 19 ist eine sechste mögliche Ausführungsform der erfindungsgemässen Notfallkapsel 510 in einer dreidimensionalen Ansicht und in Figur 20 ist die sechste Ausführungsform der Notfallkapsel 510 allerdings ohne Aufkleber gezeigt. Die Figuren 21 bis 23 zeigen die Notfallkapsel in der Draufsicht, in der Seitenansicht beziehungsweise in der Ansicht von unten. Die Notfallkapsel 510 unterscheidet sich von den Notfallkapsel 410 in der Form der Schalen 511 und 512. Das Bezugszeichen 514 kennzeichnet die Trennlinie beziehungsweise Fuge zwischen den beiden Schalen 511 und 512.

Allen Ausführungsformen der Notfallkapseln 10, 110, 210, 310, 410 und 510 ist gemeinsam, dass sich in ihrem Inneren ein flexibles Primärpackmittel 4 mit einem Stauraum 4.3 befindet. Dies ist in Fig. 5b symbolhaft dargestellt.

Eine mögliche erste Ausführungsform des flexiblen Primärpackmittels 4 (hier auch Primärverpackung genannt) ist in Figur 4a dargestellt. Bei der ersten Ausführungsform des Primärpackmittels 4 ist eine Ausnehmung 4.1 vorgesehen, die als Rissmarke dient und dem Benutzer anzeigt, an welcher Stelle das Primärpackmittel 4 am leichtesten aufzureissen ist. Zudem kann dort eine Perforation 4.2 vorgesehen sein, um das Aufreissen des Primärpackmittels 4 noch weiter zu erleichtern. Der Rand 4.4 des Primärpackmittels 4 ist bei der Ausführungsform gemäss Figur 4 verschweisst. Der Stauraum 4.3 des Primärpackmittels 4 kann verkleinert werden, indem die Luft mit Hilfe einer Vakuumier-Vorrichtung aus dem Stauraum 4.3 herausgesaugt wird. Bei Bedarf kann das Primärpackmittel zusätzlich mit Hilfe einer Pressvorrichtung zusammengepresst werden, um den Stauraum 4.3 zu verkleinern. Dies geschieht aber erst nachdem das Notfallequipment im Primärpackmittel 4 angeordnet wurde.

Eine mögliche zweite Ausführungsform des flexiblen Primärpackmittels 4 ist in Fig. 4b dargestellt. Dort ist vorgesehen, dass das Primärpackmittel 4 als Tüte ausgebildet ist, die eine Einfüllöffnung 4.5 und einen verschweissen unteren Rand 4.7 aufweist. Der linke Rand 4.6 und der rechte Rand 4.8 weisen keine Schweissnähte auf. Die Tüte kann aus einem Schlauch hergestellt werden, indem vom Schlauch ein Schlauchstück mit einer definierten Länge abgeschnitten und die Schnittkante verschweisst wird, sodass sie den unteren Rand 4.7 der Tüte bildet. Der Stauraum 4.3 des Primärpackmittels 4 kann verkleinert werden, indem die Luft mit Hilfe einer Vakuumier-Vorrichtung aus dem Stauraum 4.3 herausgesaugt wird. Bei Bedarf kann das Primärpackmittel zusätzlich zusammengepresst werden, um den Stauraum 4.3 zu verkleinern.

Unter Tüte wird hier ein hohler, dünnwandiger, leicht verformbarer Gegenstand verstanden, der geeignet ist, das Notfallequipment aufzunehmen.

Die Tüte kann aus zwei Rechtecken hergestellt werden, die an drei der vier Rändern des Rechtecks mit einander verklebt oder verschweisst sind (Fig. 4a). Statt dessen kann die Tüte auch durch ein in der Mitte gefaltetes rechteckiges Stück Stoff, Folie oder Papier gebildet werden.

Die Tüte kann auch bereits in Zylinderform (Fig. 4b) oder in Zylinderform mit Runden produziert werden. Die Rundungen können an die Innenkontur des Hohlkörpers angepasst sein. Dies hat den Vorteil, dass der zur Verfügung stehende Hohlraum 10.1 optimal ausgenutzt werden kann.

Das Primärpackmittel 4 ist vorzugsweise aus Kunststoff, zum Beispiel aus HDPE, LDPE oder biologisch abbaubarer Folie hergestellt. Sie kann aber auch aus Papier oder einem Textilmaterial sein. Das Primärpackmittel kann auch aus einem Netz hergestellt sein.

Bei einer dritten möglichen Ausführungsform des flexiblen Primärpackmittels 4 ist vorgesehen, das Primärpackmittel 4 aus einem Stück Schrumpfschlauch herzustellen. Als Schrumpfschlauch dient ein Kunststoffschlauch, der sich unter Hitzeeinwirkung zusammenzieht. Zum Erhitzen des Schrumpfschlauchs kann zum Beispiel Heissluft dienen. Auf diese Weise kann der Stauraum 4.3 des Primärpackmittels 4 verkleinert werden. Bei Bedarf kann das Primärpackmittel zusätzlich zusammengepresst werden, um den Stauraum 4.3 zu verkleinern.

Zum Befüllen der Notfallkapsel mit Notfallequipment wird in den Stauraum 4.3 des Primärpackmittels 4 das Notfallequipment 1, 2, 3 eingeführt oder eingelegt. Anschliessend wird der Stauraum 4.3 des Primärpackmittels 4 auf ein bestimmtes Packmass verkleinert. Das definierte Packmass entspricht einem Volumen, das kleiner als das Volumen des Hohlkörpers 10.1 ist. Mit anderen Worten: Der Stauraum 4.3 mit dem darin befindlichen Notfallequipment wird zuerst auf ein Volumen reduziert, das so klein ist, dass das Primärpackmittel 4 mit dem Notfallequipment im Hohlkörper 10.1 Platz findet. Nachdem der Stauraum 4.3 ausreichend stark verkleinert worden ist, behält das Primärpackmittel 4 mit dem Notfallequipment sein reduziertes Volumen bei, und zwar unabhängig davon wo es sich gerade befindet. Das so komprimierte Primärpackmittel 4 mit dem Notfallequipment kann also auch ohne den Hohlkörper aufbewahrt und zu einem beliebigen späteren Zeitpunkt in den Hohlköper beziehungsweise in die Schalen 11 und 12 gesteckt werden. Auf welche Art der Stauraum 4.3 verkleinert wird, hängt davon ab, welches Primärpackmittel 4 verwendet wird. Nähere Ausführungen hierzu finden sich in der Beschreibung weiter oben. Anschliessend wird das das Notfallequipment 1, 2, 3 enthaltende, verkleinerte Primärpackmittel 4 im Hohlkörper der Notfallkapsel 10 angeordnet. Wenn der Hohlkörper aus den beiden Schalen 11 und 12 besteht, wird das das Notfallequipment enthaltende, verkleinerte Primärpackmittel 4 in die eine der beiden Schalen 11 beziehungsweise 12 gesteckt. Anschliessend wird die gefüllte Schale mit der anderen Schale 12 bzw. 11 verschlossen. Bei Bedarf kann dann ein Aufkleber 13 auf der Aussenseite des Hohlkörpers angebracht werden.

Das Primärpackmittel 4 steht in direkten Kontakt mit dem Notfallequipment, also den Schutzgegenständen 1, 2 und/oder 3 und dient dazu, das Packmass des Notfallequipments zu verkleinern. Vorzugsweise wird das Packmass des Primärpackmittels 4 mit dem Notfallequipment gerade so weit verkleinert, dass es im Hohlraum 10.1 der Notfallkapsel 10 Platz findet.

Die Notfallkapsel 10 mit ihrem Hohlkörper beziehungsweise die beiden Schalen 11 und 12 können auch als Sekundärpackmittel angesehen werden.

Es ist auch möglich die Notfallkapsel 10 so zu gestalten, dass sie wasserdicht ist. Dazu können die beiden Schalen 11 und 12 sowie die Verbindung zwischen den beiden Schalen 11 und 12 wasserdicht ausgeführt sein. Stattdessen oder zusätzlich dazu kann auch das Primärpackmittel 4 so ausgestaltet sein, des es das Notfallequipment als wasserdichte Hülle umgibt. Dies hat den Vorteil, dass man die Notfallkapsel auch beim Baden und Schwimmen im Wasser mitführen kann. Sinngemäss das Gleiche gilt auch für die anderen der oben beschriebenen Ausführungsformen der Notfallkapsel.

Wenn das Notfallequipment im Primärpackmittel 4 vakuumiert ist, bleibt es noch besser vor äusseren Einflüssen geschützt. Auch die Hygiene wird dadurch noch weiter verbessert. Eine Verschmutzung des Notfallequipments kann dadurch noch besser vermieden werden.

Nachdem das Notfallequipment verwendet wurde, kann die leere Notfallkapsel mit neuem Notfallequipment befüllt werden. Die leere Notfallkapsel kann also mehrmals wiederbenutzt werden, was der Umwelt zugutekommt.

Die beiden Schalen 11 und 12 können zwei separate Bauteile sein, sodass sie vollständig voneinander getrennt werden können. Statt dessen können die beiden Schalen 11 und 12 aber auch über einen vorzugsweise dünnen, elastischen Steg miteinander verbunden sein (in den Figuren nicht gezeigt), sodass die beiden Schalen 11 und 12 ein einstückiges Bauteil bilden. Der elastische Steg sorgt dafür, dass nach dem Öffnen der Notfallkapsel der Kapselinhalt (das Notfallequipment) zwar zugänglich ist und herausgenommen werden kann, die beiden Schalen 11 und 12 aber nach wie vor miteinander verbunden bleiben. Damit kann weder die eine noch die andere Schale verloren gehen. Das Gleiche gilt sinngemäss auch für die Schalen 111 - 512 der weiteren Ausführungsformen.

Wie beispielsweise in Figur 7 zu sehen ist, gibt die Prägung 17 ein Kreuz wieder, das auf die Funktion der Notfallkapsel 210 als Ausstattung für die Erste Hilfe hinweist. Das Gleiche gilt sinngemäss auch für die Prägungen 117, 217 und 517 bei den anderen Ausführungsformen.

Die Schalen 111 - 512 können zum Beispiel aus Kunststoff oder Aluminium hergestellt sein.

Die vorhergehende Beschreibung der Ausführungsbeispiele gemäss der vorliegenden Erfindung dient nur zu illustrativen Zwecken. Im Rahmen der Erfindung sind verschiedene Änderungen und Modifikationen möglich. So sind beispielsweise die verschiedenen in den Figuren 4a und 4b gezeigten Komponenten des Primärpackmittels und die in den Figuren 5 bis 24 gezeigten Komponenten der Notfallkapsel auch auf eine andere als in den Figuren gezeigte Weise miteinander kombinierbar.

### Bezugszeichenliste

- 1: Handschuh
- 2: Maske beziehungsweise Beatmungsmaske
- 2.1: Filter
- 2.2: Elastikband
- 3: Anleitung
- 4: Primärpackmittel
- 4.1: Ausnehmung
- 4.2: Perforation
- 4.3: Stauraum
- 4.4: Rand
- 4.5: Einfüllöffnung
- 4.6: linker Rand
- 4.7: unterer Rand
- 4.8: rechter Rand
- 10: Notfallkapsel
- 10.1: Hohlraum
- 11: erste Schale
- 11.1: Nut
- 12: zweite Schale
- 12.1: Wulst
- 13: Aufkleber
- 14: Fuge beziehungsweise Trennlinie
- 15: Ring
- 16: Befestigung
- 17: Prägung
- 110: Notfallkapsel
- 111: erste Schale
- 111.1: Wulst
- 112: zweite Schale
- 112.1: Nut
- 113: Aufkleber
- 210: Notfallkapsel
- 211: erste Schale
- 212: zweite Schale
- 217: Prägung
- 310: Notfallkapsel
- 310.1: Hohlraum
- 311: erste Schale
- 312: zweite Schale
- 314: Fuge beziehungsweise Trennlinie
- 315: Befestigungsring
- 318: Text
- 410: Notfallkapsel
- 411: erste Schale
- 412: zweite Schale
- 413: Aufkleber
- 414: Fuge beziehungsweise Trennlinie
- 415: Befestigungsring / Aufhänger
- 510: Notfallkapsel
- 510.1: Hohlraum
- 511: erste Schale
- 512: zweite Schale
- 513: Aufkleber
- 514: Fuge beziehungsweise Trennlinie
- 515: Befestigungsring / Aufhänger

## Patentansprüche

1. Notfallkapsel mit Notfallequipment,
- bei der ein manuell öffenbarer Hohlkörper (11, 12) vorgesehen ist,
- bei der ein Primärpackmittel (4) aus flexiblem Material vorgesehen ist, das einen Stauraum (4.3) aufweist, in dem sich das Notfallequipment (1, 2, 3) befindet,
- bei der das Primärpackmittel (4) derart ausgebildet ist, dass dessen Stauraum (4.3) auf ein definiertes Packmass verkleinerbar ist und das Packmass auch ausserhalb des Hohlkörpers (11, 12) beibehält, und
- bei der das Primärpackmittel (4) samt Notfallequipment (1, 2, 3) im verkleinerten Zustand im Hohlkörper (11, 12) angeordnet ist.

2. Notfallkapsel nach Patentanspruch 1,
bei der das Primärpackmittel (4) derart ausgebildet ist, dass dessen Stauraum (4.3) durch Pressen, durch Unterdruck und/oder durch Erhitzen verkleinerbar ist.

3. Notfallkapsel nach Patentanspruch 1 oder 2,
bei der das Primärpackmittel (4) aus Kunststoff hergestellt ist.

4. Notfallkapsel nach Patentanspruch 1 oder 2,
bei der das Primärpackmittel (4) aus einem Kunststoff hergestellt ist, der bei Erwärmung schrumpft.

5. Notfallkapsel nach einem der vorherigen Patentansprüche,
bei der das Primärpackmittel (4) eine Einfüllöffnung aufweist, die verschweisst oder verklebt ist, wenn der Stauraum (4.3) mit dem Notfallequipment (1, 2, 3) befüllt ist.

6. Notfallkapsel nach einem der vorherigen Patentansprüche,
- bei der der Hohlkörper (11, 12) eine erste Schale (11) und eine zweite Schale (12) aufweist, und
- bei der die erste Schale (11) und die zweite Schale (12) formschlüssig miteinander verbindbar sind.

7. Notfallkapsel nach Patentanspruch 6,
- bei der die erste Schale (11) eine Nut (11.1) und die zweite Schale (12) einen Wulst (12.1) aufweist, und
- bei der der Wulst (12.1) und die Nut (11.1) dazu vorgesehen sind einen Formschluss zu bilden.

8. Notfallkapsel nach Patentanspruch 6,
bei der die erste Schale (11) und die zweite Schale (12) miteinander verschraubt sind.

9. Notfallkapsel nach einem der vorherigen Patentansprüche,
bei der das Notfallequipment Handschuhe (1) und eine Maske (2) umfasst.

10. Notfallkapsel nach Patentanspruch 9,
bei der das Notfallequipment eine Anleitung (3) umfasst.

11. Notfallkapsel nach einem der vorherigen Patentansprüche,
bei der ein Aufhänger (15) vorgesehen ist, der am Hohlkörper (11, 12) befestigt ist.

12. Verfahren zum Befüllen einer Notfallkapsel mit Notfallequipment nach einem der vorherigen Patentansprüche, das folgende Schritte umfasst:
- in den Stauraum (4.3) des Primärpackmittels (4), wird das Notfallequipment (1, 2, 3) eingebracht,
- der Stauraum (4.3) des Primärpackmittels (4) wird auf ein bestimmtes Packmass verkleinert und es wird dafür gesorgt, dass das Packmass erhalten bleibt,
- anschliessend wird das das Notfallequipment (1, 2, 3) enthaltende, verkleinerte Primärpackmittel (4) im Hohlkörper (11, 12) der Notfallkapsel (10) angeordnet.

13. Verfahren nach Patentanspruch 12,
bei dem der Stauraum (4.3) verkleinert wird, indem im Stauraum (4.3) ein Unterdruck erzeugt wird.

14. Verfahren nach Patentanspruch 12 oder 13,
bei dem der Stauraum (4.3) verkleinert wird, indem das Primärpackmittel (4) zusammengepresst wird.

15. Verfahren nach Patentanspruch 12, 13 oder 14,
bei dem der Stauraum (4.3) verkleinert wird, indem das Primärpackmittel (4) durch Erhitzten einem Schrumpfungsprozess unterzogen wird.

16. Verfahren nach einem der Patentansprüche 12 bis 15,
bei dem der Stauraum (4.3) luftdicht verschlossen wird.
